(19) Europäisches Patentamt
European Patent Office
Office européen des brevets

(11) Publication number: **0 304 157 A1**

# (12) EUROPEAN PATENT APPLICATION

(21) Application number: 88306402.4

(51) Int. Cl.4: **C07D 403/14 , A61K 31/475**

(22) Date of filing: 13.07.88

(30) Priority: 17.07.87 US 74978

(43) Date of publication of application: 22.02.89 Bulletin 89/08

(84) Designated Contracting States: DE ES FR GB IT

(71) Applicant: HARBOR BRANCH OCEANOGRAPHIC INSTITUTION, INC.
5600 Old Dixie Highway
Fort Pierce, FL 33450(US)

(72) Inventor: Gunasekera, Sarath P.
450 21st Ct.
Vero Beach Florida 32962(US)
Inventor: Cross, Sue S.
1915 Jacaranda Drive
Ft. Pierce Florida 33449(US)

(74) Representative: Marchant, James Ian et al
Elkington and Fife Beacon House 113 Kingsway
London WC2B 6PP(GB)

## (54) Antitumor and antiviral alkaloids.

(57) Bis-indola alkaloids of formula:

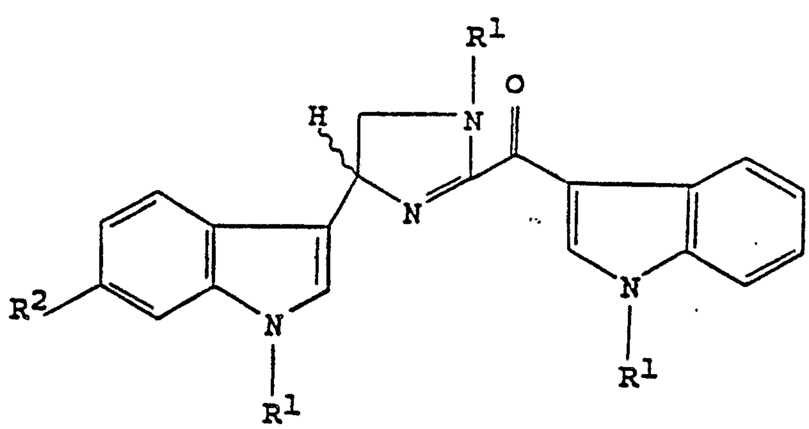

wherein $R^1$ is a hydrogen, lower alkyl, or lower acyl group; and $R^2$ is a hydrogen or halogen group, are useful for inhibiting tumors and viruses. The alkaloids are devided from marine organisms i.e. the marine sponge genus Topsentia.

EP 0 304 157 A1

# Antitumor and Antiviral Alkaloids

## Field of the Invention

This invention relates to new cyclic organic compounds which have useful antiviral and antitumor activity. More particularly, this invention relates to new bis-indole alkaloid antitumor and antiviral compositions derived from marine organisms, i.e., the marine sponge, Topsentia sp., and their methods of use.

## Background of the Invention

Various tumor related diseases inflict man. Considerable research has been devoted to oncology and antitumor measures. Tumors are common in a variety of mammals and the prevention, control of the growth and regression of tumors in mammals is important to man. The term tumor refers to abnormal masses of new tissue growth which is discordant with the economy of the tissue of origin or of the host's body as a whole.

Tumors inflict mammals and man with a variety of disorders and conditions including various forms of cancer and resultant cancerous cachexia. Cancerous cachexia refers to the symptomatic discomfort that accompanies the infliction of a mammal with a tumor. These symptoms include weakened condition of the inflicted mammal as evidenced by, for example, weight loss. The seriousness of cancer is well known, e.g., cancer is second only to heart and vascular diseases as a cause of death in man.

Considerable research and resources have been devoted to onocology and antitumor measures including chemotherapy. While certain methods and chemical compositions have been developed which aid in inhibiting, remitting or controlling the growth of tumors, new methods and antitumor chemical compositions are needed.

Viral diseases also inflict man, plants, insects and animals. The prevention and control of viral diseases has important health and economic implications.

Viral diseases contribute to inflictions in humans including common colds, herpes and cancer and the importance of their control is obvious. Also important is the control of viral diseases in animals for economic reasons as well as the ability of such animals to become virus reservoirs or carriers which facilitate the spreading of viral diseases to humans. Viral plant diseases have been known to have a disruptive effect on the cultivation of fruit trees, tobacco, and various vegetables. Insect viral diseases are also of interest because of the insects' ability to transfer viral diseases to humans.

The prevention and control of viral diseases is thus of prime importance to man and considerable research has been devoted to antiviral measures. Certain methods and chemical compositions have been developed which aid in inhibiting, controlling or destroying viruses but additional methods and antiviral chemical compositions are needed.

Marine organisms and particularly marine sponges are a potential source for chemically and biologically interesting molecules of great diversity. Some such molecules derived from sponges are described in Scheuer, P.J. Ed., Marine Natural Products, Chemical and Biological Perspectives; Academic Press, New York, 1978-1983; Vol. I-V; Faulkner, D.J. Natural Products Reports 1984, Vol. 1, 551-598; and 1986, Vol. 3, 1-33 Uemura, D.; Takahashi, K.; Yamamoto, T.; Katayama, C; Tanaka, J.; Okumura, Y.; Hirata, Y. J. Am. Chem. Soc. 1985, 107, 4796-4798

Copending application Serial no. 935,401 filed November 26, 1986 by S.P. Gunasekera, S.S. Cross, Y. Kashman and M.S. Lui discloses Topsentin compositions of the formula:

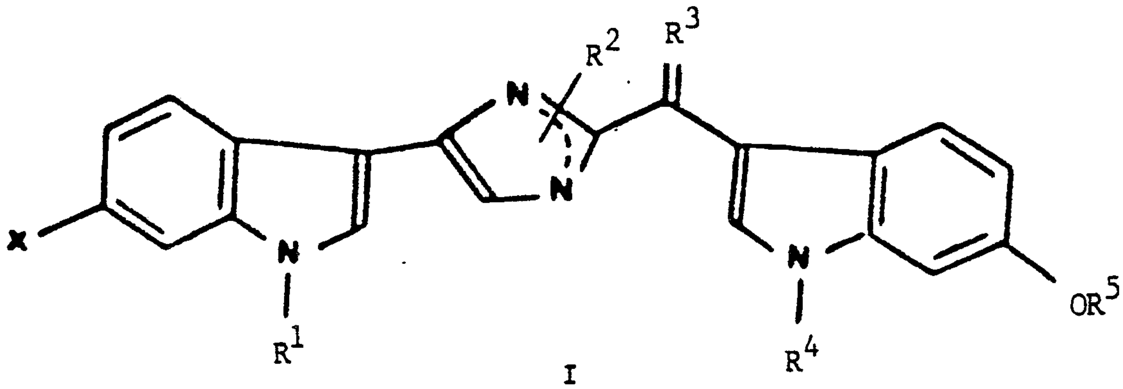

I

wherein $R^1$, $R^2$, $R^4$ and $R^5$ are the same or different and are a hydrogen, lower alkyl, or lower acyl group; $R^3$ is a double bonded oxygen or a combination of two single bonded hydrogen, hydroxy, lower alkoxy, or lower acyloxy groups which are the same or different; and X is a hydrogen, hydroxy or halogen group. These compositions are disclosed to have useful antitumor and antiviral properties. The entire disclosures of this application is hereby incorporated herein by reference.

It has now been found that certain novel bis-indole alkaloid compositions derived from extracts of the marine sponge, Topsentia, sp. possess useful antitumor and antiviral activity.

## Summary of the Invention

It is therefore an object of the invention to provide novel compositions which are useful as antiviral and antitumor agents and a process for producing such novel antitumor and antiviral compositions.

Additional objects and advantages of the invention will set forth, in part in the description which follows and in part will be obvious from this description, or may be learned by the practice of invention. The objects and advantages of the invention are realized and obtained by means of the compositions, process, methods, and combinations particularly pointed out in the appended claims.

To achieve the objects in accordance with the purposes of the invention, as embodied and fully described herein, the invention comprises compositions of the general formula (I):

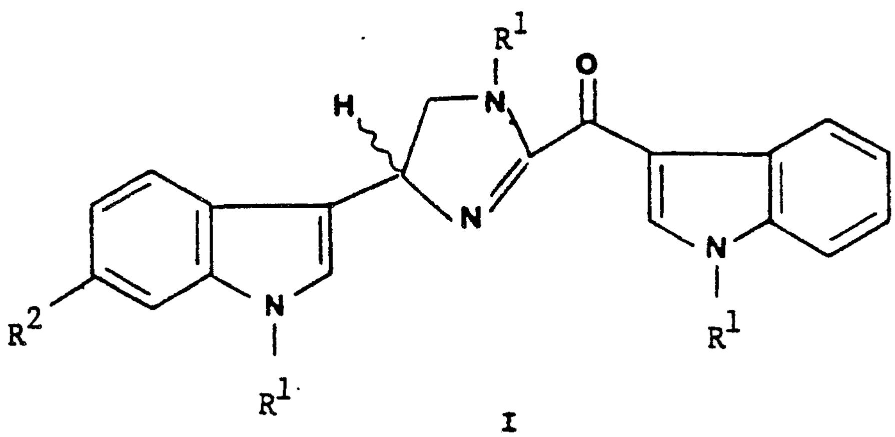

I

wherein $R^1$ is a hydrogen, lower alkyl, or lower acyl group; and $R^2$ is a hydrogen or halogen group.

In preferred embodiments of the invention, the compositions are substantially pure.

In preferred embodiments of the invention, the lower alkyl and acyl groups have from 1 to 5 carbon atoms.

In further preferred embodiments of the invention, the invention comprises a composition of the general formula (I) wherein $R^1$ and $R^2$ have the following identities:

(1) $R^1$ = H, $R^2$ = H

(2) $R^1$ = $CH_3$, $R^2$ = Br

(3) $R^1$ = $COCH_3$, $R^2$ = Br
(4) $R^1$ = $CH_3$, $R^2$ = H
(5) $R^1$ = $COCH_3$, $R^2$ = H
(6) $R^1$ = H; $R^2$ = Br

In more preferred embodiments of the invention, the invention comprises a composition of the Formula (II).

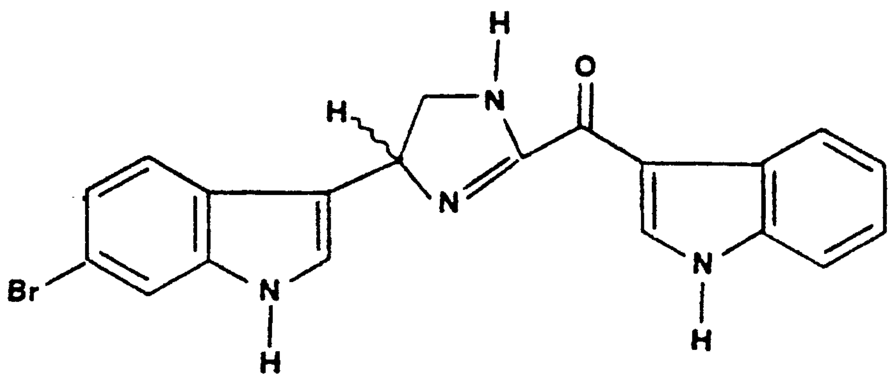

II

As embodied and fully described herein, the invention also comprises a process to produce the compositions of formulae I and II. The process comprises the steps of collecting the marine sponge Topsentia sp; contacting the sponge with at least one suitable organic solvent to obtain an extract comprising a composition according to formula I or II; and isolating a composition according to formula I or II from the extract.

In preferred embodiment of the invention, the suitable organic solvent is selected from the group consisting of, but not limited to, acetone, methanol, ethanol, propanol, isopropanol, and butanol.

As embodied and fully described herein, the invention further comprises a method for inhibiting viruses comprising contacting a virus with an effective anti-viral amount of one or more compositions according to formula I or II.

It is to be understood that both the foregoing general descriptions and the following detailed description are exemplary and explanatory only, and are not intended to be restrictive of the invention as claimed.

## Detailed Descriptions of the Performance Embodiments of the Invention

References will now be made in detail to present preferred embodiments of the invention, examples of which are illustrated in the following example section.

In accordance with the invention, novel compositions are provided to achieve the objects in accordance with the purposes of the invention, as embodied and fully described herein, the invention comprises compositions of the general formula (I):

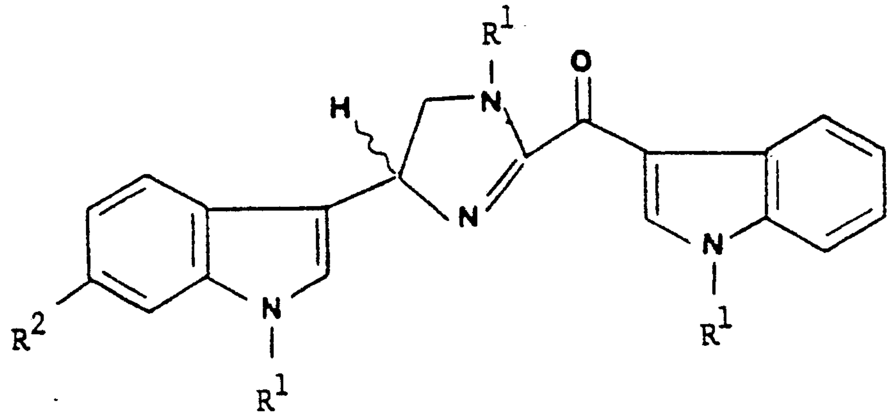

I

wherein $R^1$ is a hydrogen, lower alkyl, or lower acyl group and $R^2$ is a hydrogen or halogen group.

In preferred embodiments of the invention, the composition is substantially pure.

In preferred embodiments of the invention, the lower alkyl and acyl groups have from 1 to 5 carbon atoms.

In further preferred embodiments of the invention, the invention comprises a composition of the general formula (I) wherein $R^1$ and $R^2$ have the following identities:

(1) $R^1$ = H, $R^2$ = H
(2) $R^1$ = $CH_3$, $R^2$ = Br
(3) $R^1$ = $COCH_3$, $R^2$ = Br
(4) $R^1$ = $CH_3$, $R^2$ = H
(5) $R^1$ = $COCH_3$, $R^2$ = H
(6) $R^1$ = H; $R^2$ = Br

In more preferred embodiments of the inventions, the invention comprises a composition of the general formula (II):

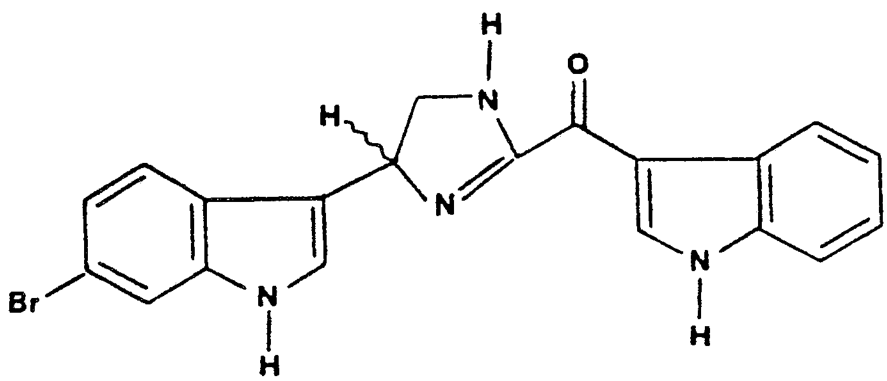

II

In accordance with the invention, an antitumor composition is provided comprising as active ingredient an effective antitumor amount of one or more of the compositions described above and identified by formulas I and II and a non-toxic, pharmaceutically acceptable carrier or diluent. While effective amounts vary, as conditions in which the antitumor compositions are used vary, a minimal dosage required for activity is generally between 0.5 and 100 micrograms against $10^5$ tumor cells. Useful examples of non-toxic pharmaceutically acceptable carriers or diluents include, but are not limited to the following: ethanol, dimethyl sulfoxide and glycerol.

In accordance with the invention, a method for inhibiting tumors in a host is provided comprising contacting a tumor with an antitumor amount of one or more compositions according to formulae I or II. The effectiveness of the compositions of the invention for inhibiting tumors and tumor cells indicates their usefulness for controling rumors in hosts, including mammals, and for treating cancerous cachexia.

In accordance with the invention, an antiviral composition is provided comprising as active ingredient an effective antiviral amount of one or more of the compositions described above and identified by formula I or II and a non-toxic pharmaceutically acceptable carrier or diluent. While effective amounts may vary, as conditions in which the antiviral compositions are used vary, a minimal dosage required for activity is generally between 50 and 200 micrograms against 25 -100 infectious doses ($ID_{50's}$ of virus. Useful examples of non-toxic pharmaceutically acceptable carriers or diluents include, but are not limited to, the following: ethanol; dimethyl sulfoxide; and glycerol.

In accordance with the present invention, virus cells are inhibited or killed by a method comprising contacting a virus with an effective antiviral amount of one or more compositions according to formulae I or II. The minimal effective amount as stated above is generally from 50 to 200 micrograms against 25 to 100 $ID_{50's}$ of virus. The compositions of formulae I or II are active for inhibiting or killing a diverse range of viruses including, but not limited to, the RNA viruses, vesicular stomatitis (herein "VSV"), arenaviruses, coronaviruses, rhinoviruses, influenza viruses and the DNA viruses, herpes simplex-I (herein "HSV-I"), other herpes viruses, adenoviruses, coxsackie viruses, polioviruses and papovaviruses.

The effectiveness of the compositions of the invention for inhibiting virus cells indicates that the compositions of formulae I or II should also be useful in controlling viral infections in host animals and plants which are caused by a virus which is thus inhibited or destroyed. Viral infections which may be controlled by utilizing compositions of the present invention include, but are not limited to, those caused by those RNA viruses and DNA viruses described above. The invention may also be useful in controlling common viral infections of plants.

In accordance with the invention, a process to produce compositions according to formulae I or II comprises the steps of: collecting the marine sponge; contacting the sponge with at least one suitable organic solvent to obtain an organic extract comprising a composition according to formula I or II; and isolating a composition according to formula I or II.

A detailed description and explanation of a preferred embodiment of the process of the invention to produce the compositions according to formula I or II and their reduced or acid salt derivatives is as follows: the marine sponge Topsentia, sp. is collected by SCUBA or submersible at Goulding Cay, Bahamas. The marine sponge is extracted with methanol-toluene (10:1) as a first solvent to obtain an extract which is concentrated to yield a crude residue. The residue is then partitioned between pentane and 10% aqueous methanol as first partitioning solvents to obtain an aqueous methanol extract. The alcohol layer was concentrated and re-partitioned between butanol and water. The butanol soluble fraction which comprises a composition according to formula I or II is dissolved in 20% aqueous methanol and the substantially pure composition of formula I or II is obtained by various chromatographic methods.

While methanol-toluene is the presently preferred choice for the first extracting solvent, other suitable solvents may be substituted. A suitable solvent should be capable of extracting a compound according to formula I or II from other components of the marine sponge. Acetone, ethanol, propanol, isopropanol and butanol are petitioning solvents presently used, but other solvents may be substituted.

any suitable fractionation and isolation technique may be utilized in accordance with the process of the invention. Suitable fractionation techniques include various chromotography techniques such as reverse phase high pressure liquid chromatography with a suitable column as would be known to those skilled in the art. Suitable columns include IBM reversed phase C-18. These columns are eluted with suitable eluents such as: water-ethanol; water-butanol; water-isopropanol; water-acetone; water-acetonitrile; and various combinations and ratios thereof as would be known to those skilled in the art.

## EXAMPLE

The invention will now be illustrated by example. The example is not intended to be limiting of the scope of the present invention. In conjunction with the detailed and general description above, the example provides further understanding of the present invention and outlines a process for producing compositions of the invention.

The following example represents a preferred embodiment of the compositions, processes and methods of the invention for satisfying the stated objects of the invention. The starting materials and reagents in the example whose method of preparation are not indicated are commercially available from sources known to the art such as chemical supply houses.

EXAMPLE 1

The antitumor and antiviral bis-indole alkaloids of the invention were prepared from a marine sponge, Topsentia, sp., according to the following procedure.

Preparation of 4,5-Dihydro-6″deoxybromotopsentin

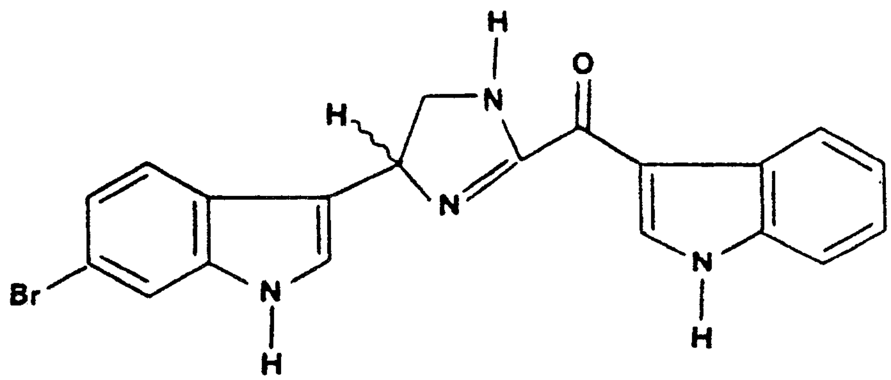

Composition 1: 4,5-Dihydro-6″-deoxybromotopsentin

A frozen sample of the marine sponge was collected at Goulding Cay, Bahamas at a depth of 756 feet using a submersible vessel.

An extract of the sponge was prepared by homogenizing the frozen organism and repeatedly steeping with methanol and 10% toluene followed by methanol. The concentrated extract was partitioned between pentane and 10% aqueous methanol. The alcohol layer was concentrated and re-partitioned between butanol and water. The butanol soluble fraction (1.42 g) was subjected to vacuum chromatography over reversed phase material [Amicon silica C-18, 20 - 45 um] using 20% aqueous methanol and separated into several fractions. The yellow colored fraction was then separated by reversed phase, high-pressure liquid chromatography (RP HPLC) (5 um, C-18, 20% $H_2O$ - MeOH) to give crude compound 1, which on re-chromatography (HPLC) furnished pure 4,5 dihydro - 6″ - deoxybromotopsentin 1, (20 mg) as a yellow powder. The physical and spectral characteristics are listed below: $[\alpha]_D^{20}$ = -170 (C=2.0 MeOH).

MS: HREI 406.027832 for $C_{20}H_{13}{}^{81}BrN_4O$ ($M^+$-2H) (Δ2.6) 404.030029 for $C_{20}H_{13}{}^{79}BrN_4O$ ($M^+$-2H) (Δ2.8)

UV: MeOH: λ max(nm), 196 (ε = 102,100), 215 (76,000), 276 sh (89,300), 294 (109,600) and 330 (7600).

IR: KBr; $cm^{-1}$ 3620,3390,3280,2920, 2860, 1665, 1570, 1450, 1420, 1332, 1240, 1160, 1120, 1100, 1020, 950, 805, and 750.

NMR:

$^1H$ DMSO-$d_6$ (360 MHz)δ11.52 (1H, bs, H-1″), 11.13 (H, bs, H-1′), 8.47 (1H, bs, H-3), 8.38 (1H, bs, H-2″), 8.37 (1H, ddd, J=8.0, 1.0, 0.7 Hz, H-4″), 7.67 (1H, d, J=8.5 Hz, H-4′), 7.58 (1H, d, J=1.7 Hz, H-7′), 7.42 (1H, ddd, J=8.0, 1.0, 0.7 Hz, H-7″), 7.29 (1H, bs, H-2′), 7.13 (1H, ddd, J=8.0, 8.0, 1.0 Hz, H-6″), 7.12 (1H, dd, J=8.5, 1.7 Hz, H-5′), 7.02 (1H, ddd, J=8.0, 8.0, 1.0 Hz, H-5″), 5.23 (1H, dd, J=9.5, 4.7 Hz, H-5), 3.60 (1H, ddd, J=12.1, 4.6, 4.4 Hz, H-4), 3.45 (1H, ddd, J=12.1, 9.5, 2.1 Hz, H-4).

$H^1$ $CD_3OD$ (360 MHz)δ 8.47 (1H, ddd, J=8.0, 1.0, 0.7 Hz, H-4″), 8.39 (1H, S, H-2″), 7.64 (1H, d, J-8.5 Hz, H-4′), 7.58 (1H, d, J=1.7 Hz, H-7′), 7.44 (1H, ddd, J=8.0, 1.0, 0.7 Hz, H-7″), 7.58 (1H, d, J=1.7 Hz, H-7′), 7.44 (1H, ddd, J=8.0, 1.0, 0.7 Hz, H-7″), 7.28 (1H, dd, J=0.9 Hz, H-2″), 7.20 (1H, ddd, J=8.0, 8.0, 1.0 Hz, H-6″), 7.18 (1H, dd, J=8.5, 1.7 Hz, H-5″), 7.12 (1H, ddd, J=8.0, 8.0, 1.0 Hz, H-5″), 5.30 (1H, ddd, J=7.0,

4.7, 0.9 Hz, H-5), 3.75 (1H, dd, J=8.7, 4.7 Hz, H-4), 3.59 (1H,dd, J=8.7, 7.0 Hz, H-4).
$^{13}C$ $CD_3OD$ (90 $MH_z$),δ 160.46 (s), 159.09 (s), 139.29 (s), 138.04 (s), 132.94 (d), 127.54 (s), 126.42 (s), 124.66 (d), 123.73 (d), 123.60 (d), 123.04 (d), 121.94 (d), 121.48 (d), 116.14 (s) 116.04(s), 115.34 (d), 112.71 (s), 112.36 (d), 55.52 and 45.03 (t).

## Antitumor Activities of the Compositions of the Invention

The assay method was utilized to illustrate the antitumor effectiveness of the compositions of formulae I and II, corresponding to composition 1 (4,5-Dihydro-6″ deoxybromo-topsentin) of the example.

## P388 MOUSE LEUKEMIA CELL ASSAY

### Maintenance of Cell Line

P388 mouse leukemia cells are grown in Dulbecco MEM medium with 10% horse serum, 1-glutamine, and 20ug/ml gentamycin (Biologos, Inc.). Cells are incubated in 10% $CO_2$ and subcultured 2 times per week.

### PROCEDURE

1. Add compound to each well of a 24-well plate or tube and allow solvent to evaporate to dryness.
2. Add 2ml (1.2 x $10^5$) cells to each well or tube and mix.
3. Incubate in 10% $CO_2$ at 37 for 48 hours.
4. Screening plates were read with an inverted microscope, scoring activity from 1+ to 4+ as follows: ND(not detectable), > 90%; 1+, 75-90%; 2+, 50-74%; 3+, 25-49%; 4+, < 25% of control growth. The concentration of the drug which inhibited 50% of cell replication was determined as described above except cell counts were done with the use of the Coulter counter and values compared to cell control to obtain results for 50% inhibitory concentration ($ICC_{50}$).

The results of this assay are provided below in Table 1:

Table 1

Antitumor results against P388 mouse leukemia cells.
Composition 1: P388 $IC_{50}$ = 4.0 ug/ml.

## ANTIVIRAL ACTIVITIES OF THE COMPOSITIONS OF THE INVENTION

The following assay method was utilized to demonstrate the *in vitro* antiviral effectiveness of the compositions of formulae I and II, corresponding to composition 1 (4,5-Dihydro-6″-deoxybromotopsentin) of the examples.

### Antiviral Assay for Mouse Coronavirus A-59

A. Cell Culture

1. NCTC clone 1469, a derivative of mouse liver, ATCC No. CCL 9.1$_8$ freeze 2518, paggage no. 16.
2. Received at SeaPharm January 1985, assigned log #HB/SP 16.

B. Maintenance of Cell Culture

1. Subcultures are prepared in this laboratory by brief exposure to trypsin-EDTA mixture and shaking the flask hard to remove the cells.
2. Trypsinization
   a. Aseptically remove the medium.
   b. Add 4 ml of trypsin-EDTA mixture to a 150cm$^2$ flask. Reduce volume if flasks are smaller.
   c. Leave for one minute or less and them shake flask.
   d. Add 10 ml of growth medium (Section E) and break up cell clumps with pipetting.
   e. Count cells.
3. Subcultures for maintenance of cells for assays.
   a. Seed 150 cm$^2$ tissue culture flasks with 10 x $10^6$ cells in 40 ml growth medium.
   b. Split cultures a minimum of twice a week.

C. Virus

1. Mouse hepatitis virus strain MHV-A59 classified as a coronavirus, ATCC No. 764.
2. Received January, 1985, passed in NCTC 1469 and assigned HB/SP 30 log number.

D. Preparation of Plates for Viral Assays

1. Cell concentration
   a. Dilute the cells with growth medium between 5 x $10^5$ and 7.5 x $10^5$ cells per ml.
   b. Seed 24 well trays with 1.0 ml per well.
   c. In order for test to work and be easy to read the cell concentration must be heavy.
   d. Incubate at 37 C with 5% $CO_2$.
   e. The cells must be used in the next 24 hours for viral assays. If the cells are held for a longer period of time cells begin to shed into the medium and the CPE and cell fusion takes 48 hours and longer instead of 12 to 14 hours. Assay is not sensitive and results are not comparable for 24 and 48 hours.

E.Viral Assay

1. Dilute drug extract for test in the appropriate solvent.
2. Add 20 lambda to a 12 mm by 75 mm glass tube for a 16 mm test well.
3. Allow the solvent to evaporate under the laminar flow hood.
4. Dilute the MHV-A59 in Dulbecco's phosphate buffered saline with $Ca^{++}$ and $Mg^{++}$ to the appropriate predetermined dilution for the lot number currently in use. Normally the dilution of virus in a titration which gives a 3+ to 4+ CPE in 24 hours is the dilution used in this assay.
5. Remove medium from wells of 24 well plate containing NCTC 1469 cells seeded 24 earlier.
6. Add 200 lambda of diluted virus to each test well. Add PBS to control wells.
7. Incubate cells and virus for 1 hour 37 C.
8. Pour off supernatant at end of incubation period.
9. To each glass tube add 10 lambda of dimethyl sulfoxide (DMSO).
10. Add 1 ml of maintenance medium to each glass tube.
11. Pour the contents of the glass tube into the corresponding well of the tissue culture plate.
12. Incubate infected cells at 37 C and read the following day.
13. At twelve hours areas of cell fusion are quite apparent and can be dected both vidually and microscopically.

14. At 24 hours the CPE is extensive and on strained plates the difference between activity and none is apparent from visual examination.

15. To stain plates discard medium and to each 16 mm well add 200 lambda of methylene blue stain or other appropriate stain.

16. Leave the stain on the cell sheet for 30 minutes more.

17. Pour off the stain and wash plates in tap water until the water is clear.

18. Allow the plates to dry. If it is desired the plates can be kept as a permanent record for the experiment record for the experiment.

19. Scoring drug activity

a. Cytotoxicity scoring

100% = complete cell destruction
75% = partial cell destruction
50% = partial cell destruction
25% = partial cell destruction
0% = no cytoxicity

b. Antiviral activity is scored from 0 to + + +.

+ + + = complete inhibition of cytopathic effects and cell fusion
+ + = partial inhibition
+ = partial inhibition
+/- = marginal inhibition
0 = no protection

F. Media

1. Growth medium for NCTC 1469 cell line

Medium NCTC 135 (GIBCO Cat. #320-1350))
10% horse serum
2% 200 mM 1-glutamine
1% nonessential amino acids (NEAA) (100X)
1% sodium pyruvate (110 mg/liter) (100X)
gentamicin 10 mg/ml (use 50 ug/ml or 0.5 ml/100
50 mg/ml (use 50 ug/ml or 0.1 ml/100

2. Maintenance medium for viral assay

Dulbecco's modified Eagle medium @4500 mg/L glucose
GIBCO Cat #320-1965
5% fetal bovine serum (FBS)
2% 200 mM 1-glutamine
1% nonessential amino acids (NEAA) (100X)
1% sodium pyruvate (110 mg/liter) (100X)
gentamicin (use 50 ug/ml)

4. Stain

Methylene Blue-certified
Sigma No. M 9140 ordered in 25 g quantity
50% ethanol:water
5 grams methylene blue/liter

This stain does not have to be filtered. It is highly soluble in this water alcohol mixture.

The results of the above antiviral assay are summarized in Table 2.

Table 2:

| Antiviral Activity against A59 mouse corona virus | | | |
|---|---|---|---|
| Composition | Dose (ug/well) | Cyt. | AVA |
| 1 | 20 | 0 | + + + |

Tables 1 and 2 show that composition 1 has antitumor and antiviral activity at concentrations of as little as 4\ug/ml and/or 20 ug/ml.

It is apparent from the in vitro testing that the compositions of the invention are effective for inhibiting viral growth and therefore for controlling viral related diseases such as herpes and the common cold, in fulfillment of the objects of the invention.

The scope of the present invention is not limited by the description, examples, and suggested uses herein and modifications can be made without departing from the spirit of the invention. For example, it may be noted that other derivatives of the compositions of example 1 such as acyl or fluorinated derivatives may possess antitumor and antiviral activity analogous to those preferred embodiments described herein may have other useful applications such as, for example, analgesic applications. Application of the compositions of the present invention can be accomplished by any suitable therapeutic method and technique presently or prospectively known to those skilled in the art. Thus, it is intended that the present invention cover the modifications and variations of this invention provided that they come within the scope of the appended claims and their equivalents.

## Claims

1. A compound of formula:

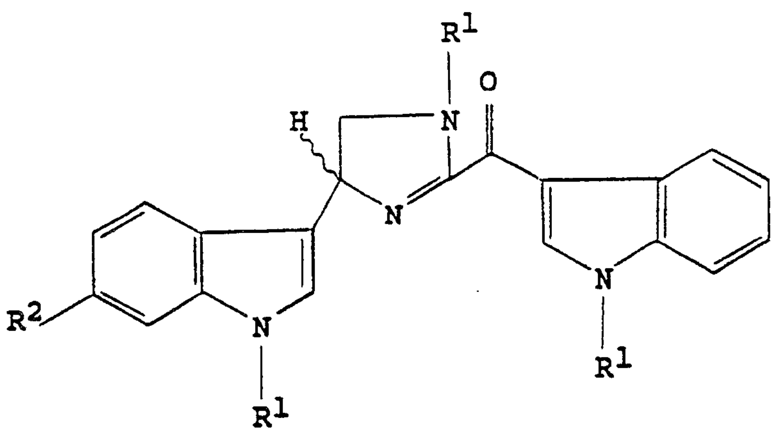

characterised in that $R^1$ is a hydrogen, lower alkyl, or lower acyl group; and $R^2$ is a hydrogen or halogen group.

2. A compound as claimed in claim 1 characterised in that $R^1$ and $R^2$ have the following identities:

(1) $R^1$ = H; $R^2$ = H
(2) $R^1$ = $CH_3$; $R^2$ = Br
(3) $R^1$ = $COCH_3$; $R^2$ = Br
(4) $R^1$ = $CH_3$; $R^2$ = H
(5) $R^1$ = $COCH_3$; $R^2$ = H
(6) $R^1$ = H; $R^2$ = Br

3. A compound as claimed in claims 1 or 2 of the formula:

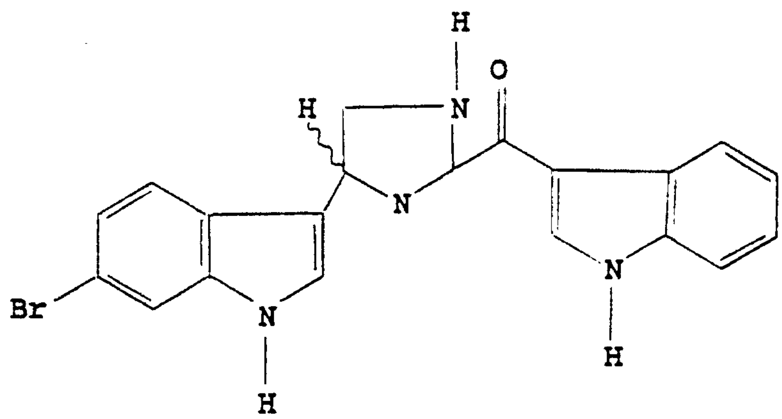

4. A compound as claimed in any of claims 1 to 3 characterised in that the compound is substantially pure.

5. An antitumor composition characterised in that the composition comprises, as active ingredient, one or more of the compounds of any of claims 1 to 4 and a non-toxic pharmaceutically acceptable carrier or diluent.

6. An antiviral composition characterised in that the composition comprises, as active ingredient, an amount of one or more of the compositions of any of claims 1 to 4 and a non-toxic pharmaceutically acceptable carrier or diluent.

7. A method for inhibiting viruses in a host characterised in that the method comprises contacting a virus with an amount of one or more of the compounds as claimed in any of claims 1 to 4.

8. A process for the production of a compound as claimed in any of claims 1 to 4 characterised in that the method comprises collecting the marine sponge, Topsentia; contacting the sponge with a suitable organic solvent to obtain an extract of the sponge and a solvent mixture; and isolating the compound as claimed in any of claims 1 to 4 from the extract.

European Patent Office

# EUROPEAN SEARCH REPORT

Application Number

EP 88 30 6402

## DOCUMENTS CONSIDERED TO BE RELEVANT

| Category | Citation of document with indication, where appropriate, of relevant passages | Relevant to claim | CLASSIFICATION OF THE APPLICATION (Int. Cl.3) |
|---|---|---|---|
| A | TETRAHEDRON LETTERS volume 27, no. 28, 1986; pages 3283-3284; G. LIDGREN et al.: "Studies of Swedish marine organisms VII. A novel biologically active indole alkaloid from the sponge Geodia Baretti" --- | | C 07 D 403/14<br>A 61 K 31/475 |
| P,A | CANADIAN JOURNAL OF CHEMISTRY volume 65, no. 9, 1987, pages 2118-2121; K. BARTIK et al.: "Topsentins, new toxic bis-indole alkaloide from the marine sponge Topsentia jenitrix" * whole document * ----- | 1,8 - | |
| | | | **TECHNICAL FIELDS SEARCHED (Int. Cl.3)** |
| | | | C 07 D 403/00<br>A 61 K 31/00 |

The present search report has been drawn up for all claims

| Place of search | Date of completion of the search | Examiner |
|---|---|---|
| BERLIN | 18-11-1988 | VAN AMSTERDAM L.J.P. |

**CATEGORY OF CITED DOCUMENTS**

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document